Europäisches Patentamt

European Patent Office

Office européen des brevets

(11) Publication number: **0 143 480**
**A2**

## (12) EUROPEAN PATENT APPLICATION

(21) Application number: **84201444.1**

(22) Date of filing: **09.10.84**

(51) Int. Cl.⁴: **A 61 K 7/043**

(30) Priority: **27.10.83 IT 2349783**

(43) Date of publication of application: **05.06.85**
**Bulletin 85/23**

(84) Designated Contracting States: **AT BE CH DE FR GB IT LI LU NL SE**

(71) Applicant: **De Vita, Claudio Giuliano, 6, Via Passeroni, I-20149 Milano (IT)**

(72) Inventor: **De Vita, Claudio Giuliano, 6, Via Passeroni, I-20149 Milano (IT)**

(74) Representative: **de Pasquale, Carlo, Via Carlo Ravizza 53, I-20149 Milano (IT)**

(54) **Nail varnish.**

(57)  A nail varnish having as a base vehicle consisting of a resin in aqueous solution, preferably a polyurethane polymer based on polyethers of polyesters with aliphatic or aromatic isocyanates, possibly extended with amines or polyglycols and suitably pigmented, which once hardened may be removed without requiring solvent, other means or substances, merely pulling it away from nail as a film after having peeled off one of its edges.

EP 0 143 480 A2

0143480

- 1 -

"NAIL VARNISH"

The presently known nail enamels consist of a solution in a solvent of synthetic resins, generally nitrocellulose, suitably pigmented with dyes of various colors and shades, which is being solidified on the nail by solvent evaporation.

Such a kind of enamel has some drawbacks, namely the troublesome smell left by the solvent in the evaporation stage during its application and the need, when the enamel is even partially deteriorated on one or more fingers for any reason, to use again the solvent to remove the residual enamel with the same troublesome condition.

During this operation, in addition again to the troublesome exhalation of the solvent, it frequently happens that, when removing enamel from one nail by said solvent, the solvent may fall on or touch nails of other fingers, so that one is compelled to apply the enamel again also on the latter.

The object of the present invention it to provide a nail varnish of novel conception and formulation, which totally removes the above indicated drawbacks, and at the same time has the required properties of adhesion, hardness, application speed, washability and gloss in the same degree or even higher than those of the known enamels.

The nail varnish of the present invention consists of a vehicle in an aqueous dispersion, thus without troublesome and harmful exhalations and for its removal requires neither solvent nor any other foreign substance, as it can be removed as a slight film, peeling off an edge with a finger nail of the other hand and then pulling said edge.

Said results were obtained by using as basic element for the preparation of the nail varnish, a polyurethane polymer in aqueous solution on the basis of polyethers or polyesters with aliphatic or aromatic isocyanates, possibly extended with amines or polyglycols.

Therefore the nail varnish according to the present invention may be applied on the nails in any place and condition, without annoying the user and/or those present with bad smell; once dried, it is similar to the known enamels as to gloss, hardness and adhesion; it resulted also to be particularly resistant to washing with water and detergents; it dries quickly, as it is possible to wash hands already ten minutes after its application, that is a time which is even less than that required with known enamels; it resulted also to have a considerable elasticity, contrary to brittleness and stiffness of known enamels; it may be produced in any color, from transparent neutral to colors of any desired shade and pearly iridescent hues; finally for its physical characteristics it may be removed in any place or moment without requiring the aid of solvents, other means or substances, but merely after having peeled off and edge and removing the rest pulling it away as a film.

Some preferred embodiments of the nail varnish according to the present invention are given hereinafter as non limiting examples only.

EXAMPLE 1

| | |
|---|---|
| - Polyurethane polymer based on polyester and aliphatic isocyanate, e.g. Neorez R974 of Polyvinyl Chemie in aqueous dispersion | 100 parts |
| - Organic dyestuff e.g. Luconyl red 3855 of BASF | 2 ÷ 5 parts |

EXAMPLE 2

| | |
|---|---|
| - Polyurethane polymer based on polyester and aliphatic isocyanate in aqueous dispersion, e.g. Neorez R 974 | 100 parts 100 parts |
| - Fluorocarbon acrylic copolymer in aqueous dispersion, e.g. Soleproof B of Dupont De Nemours | 5 parts |
| - Surfactant, e.g. Triton X 405 of Rohm & Haas | 0.1 parts |
| - Concentrated organic dyestuff | 2 ÷ 5 parts |

This type proved to be particularly well suited for its resistance to hot water and detergents.

EXAMPLE 3

| | |
|---|---|
| - Polyurethane polymer based on polyester and aliphatic isocyanate, e.g. Neorez R974 of Polyvinyl Chemie in aqueous dispersion | 100 parts |
| - Dispersion of polyethylene or polypropylene or fluorocarbon waxes, e.g. Cerfobol R/75 F.lli Lamberti | 5 parts |
| - Dyestuff | 2 ÷ 5 parts |

In case of need, where use or market require it, the products made with the above indicated compositions may be thickened by known cellulosic derivatives, e.g. Tilose of

0143480

Bayer AG.

Going thoroughly into research and development, it has also been found that several other polymers in aqueous dispersion might be used for preparing a nail varnish according to the invention, namely acrylic-chlorovinyl resins, copolymers of vinyl acetates, fluorinated copolymers and so forth even if these products do not have presently such properties of resistance and hardness and/or elasticity as those set forth in the examples; nonetheless, when suitably mixed with polyurethane resins or other substances known to a man skilled in the art, they may improve considerably these characteristics and therefore also the use of said resins as nail varnish is to be intended as falling in the scope of the invention.

It was indeed found that excellent products may be obtained at a cost lower than that of the preceding examples, by mixing polyurethane resins in aqueous dispersion, with acrylic resins in aqueous dispersion, as shown in the following example.

EXAMPLE 4

- Polyurethane polymer based on polyester
  with aliphatic isocyanate, e.g. Neorez R 974 of
  Polyvinyl Chemie in aqueous dispersion                    70 - 80 parts
- Acrylic polymer, e.g. Neocryl XK 51 of
  Polyvinyl Chemie in aqueous dispersion                    30 - 20 parts
- Dyestuff                                                  2 ÷ 5 parts

- 1 -

0143480

## CLAIMS

1) Nail varnish comprising a base vehicle consisting of a resin in aqueous solution which, once hardened, may be removed without solvents, other means or substances, merely pulling it away from nail as a film.

2) Nail varnish according to Claim 1, wherein the basic resin is an aqueous dispersion of a polyurethane polymer based on polyethers or polyesters with aliphatic or aromatic isocyanates, possibly extended with amines or polyglycols and suitably pigmented.

3) Nail varnish according to Claim 1 and 2, wherein the basic resin may be pigmented so as to be of any color, from transparent neutral to any desired shade and hue, including pearly iridescent and fluorescent hues.

4) Nail varnish according to Claims 1, 2 and 3 comprising 100 parts of polyurethane polymer based on polyester and aliphatic isocyanate in aqueous dispersion, e.g. Noarez R 974 of Polyvinyl Chemie, and 2 ÷ 5 parts of an organic dyestuff, e.g. Luconyl red 3855 of BASF.

5) Nail varnish according to Claims 1, 2 and 3 comprising 100 parts of a polyurethane polymer based on polyester and aliphatic isocyanate in aqueous dispersion, e.g. Neorez R 974, 5 parts of an acrylic fluorocarbon copolymer in aqueous dispersion, e.g. Soleproof B of Dupont De Nemous; 0.1 parts of a surfactant, e.g. Triton X 405 of Rohm & Haas and 2 ÷ 5 parts of a concentrated organic dyestuff.

6) Nail varnish according to Claims 1, 2 and 3 comprising 100 parts of a polyurethane polymer based on polyester and aliphatic isocyanate in aqueous dispersion, e.g. Neorez R 974 of Polyvinyl Chemie, 5 parts of a dispersion of polyethylene, polypropylene or fluorocarbon waxes, e.g. Cerfobol R/75 of F.lli Lamberti and 2 ÷ 5 parts of dyestuff.

0143480

7) Nail varnish according to Claim 1, 2 and 3, wherein the composition comprises acrylic resins, chlorovinyl resins, copolymers of vinyl acetate, fluorinated copolymers in aqueous dispersion, mixed with substances increasing their hardness and/or elasticity, more particularly polyurethane resins in aqueous dispersion.

8) Nail varnish according to Claim 7, comprising 70 - 80 parts of a polyurethane polymer based on polyester and aliphatic isocyanate is aqueos dispersion, e.g. Neorez of Polyvinyl Chemie, 30 - 20 parts of an aqueous dispersion of acrylic resins, e.g. Neocryl XK 51 of Polyvinyl Chemie, and 2 ÷ 5 parts of dyestuff.